# FASCICULE DE BREVET EUROPEEN

(11) **EP 1 501 783 B1**
(45) Date de publication et mention de la délivrance du brevet: **16.01.2019**
(21) Numéro de dépôt: 03740664.2
(22) Date de dépôt: 24.04.2003
(51) Int. Cl.: C07C 69/52, C07C 67/03, C07C 67/08, C07C 69/28, C07C 69/30, C07C 69/33, C07C 69/58, C11C 3/00, C11C 3/06

(54) **PROCEDES DE TRANSESTERIFICATION, ESTERIFICATION, INTERESTERIFICATION, PAR CHAUFFAGE DIELECTRIQUE**
VERFAHREN ZUR UMESTERUNG UND VERESTERUNG UNTER DIELEKTRISCHER ERWÄRMUNG
TRANSESTERIFICATION, ESTERIFICATION, INTERESTERIFICATION METHODS USING DIELECTRIC HEATING

(30) Priorité: 25.04.2002 FR 0205396
(43) Date de publication de la demande: 02.02.2005
(73) Titulaire: Aldivia S.A., 69561 Saint-Genis Laval Cedex (FR)
(72) Inventeur: CHARLIER DE CHILY, Pierre, F-60540 Irigny (FR); RAYNARD, Mikaële, F-44250 Saint-Brévin-Les-Pins (FR)
(74) Mandataire: Lavoix
(86) Numéro de dépôt international: PCT/FR2003/001307
(87) Numéro de publication internationale: WO 2003/090669

(56) Documents cités:
- EP-A- 0 884 305
- EP-A2- 1 256 565
- WO-A2-03/014272
- GB-A- 2 361 918
- US-A- 5 387 397
- US-A- 5 585 506
- DATABASE WPI Section Ch, Week 197727 Derwent Publications Ltd., London, GB; Class E17, AN 1977-48323Y XP002226439 & SU 528 300 A (SHEKHTER YU N), 15 octobre 1976 (1976-10-15)
- LIDSTROM P ET AL: "Microwave assisted organic synthesis-a review", TETRAHEDRON, ELSEVIER SCIENCE PUBLISHERS, AMSTERDAM, NL, vol. 57, no. 45, 5 November 2001 (2001-11-05), pages 9225-9283, XP004312089, ISSN: 0040-4020, DOI: 10.1016/S0040-4020(01)00906-1

## Description

### Secteur technique de l'invention et problème posé :

Les alcools polyhydroxylés partiellement ou totalement estérifiés sont très recherchés et utilisés en cosmétique, dermatologie, alimentaire, pour les lubrifiants, les plastiques, les produits phytosanitaires...

En cosmétique, par exemple, les esters sont des composés très importants parce qu'ils représentent une forte proportion des matières premières utilisées dans les formulations. Ils jouent le rôle de conditionneur, d'émollient et d'émulsionnant.

Les esters peuvent être obtenus par estérification, intérestérification ou transestérification, procédés connus de l'homme de l'art.

La présente invention concerne des procédés nouveaux d'estérification, transestérification et intérestérification. Ces nouveaux procédés permettent non seulement de diminuer les temps réactionnels et ils conduisent également à l'obtention d'esters aux caractéristiques physico-chimiques remarquables : ces procédés permettent en effet de réduire de manière significative l'indice d'acide et l'indice de peroxyde du mélange.

### Art antérieur :

### Définition d'un ester

Un ester a pour formule chimique générale R-COOR' provenant d'une fonction acide RCOOH et d'une fonction alcool R'OH.

### Réactions chimiques

Les esters peuvent être obtenus par estérification, intérestérification ou transestérification.

### a- l'estérification

L'estérification est une réaction réversible et limitée (non totale) d'un acide carboxylique sur un alcool. La réaction est athermique et très lente. Elle nécessite l'emploi d'un catalyseur acide puissant comme l'APTS (Acide Para-Toluène Sulfinique).

RCOOH + R'OH ⇔ RCOOR' + H2O

La fin de la réaction est un état d'équilibre où les quatre constituants sont présents dans des proportions qui ne varient plus au cours du temps. La limite d'estérification est déterminée par la classe de l'alcool. A partir d'une mole d'acide et d'une mole d'alcool, on obtient :
- 0.65 mole d'ester si l'alcool est primaire
- 0.60 mole d'ester si l'alcool est secondaire
- 0.05 mole d'ester si l'alcool est tertiaire.

La température ne modifie pas la composition du mélange en équilibre mais une élévation de température permet d'atteindre plus vite cet état d'équilibre.

Pour activer la réaction et la déplacer dans le sens de la formation de l'ester, il faut :
- éliminer au fur et à mesure l'eau qui se forme en créant un azéotrope avec un solvant, tel que le benzène, et opérer à la température d'ébullition du mélange
- ajouter un excès d'un des réactifs
- opérer en présence d'un catalyseur acide
- ou remplacer l'acide par l'un de ses dérivés : anhydride ou chlorure d'acide. Dans ce cas, il convient d'ajouter de la pyridine et un catalyseur acide au mélange réactionnel.

Il est également possible d'utiliser des catalyseurs enzymatiques pour cette réaction. L'estérification enzymatique présente de nombreux avantages : elle permet de travailler à faible température et d'obtenir un meilleur rendement (90%). De plus, les lipases sont supportées et ne laissent aucune trace dans l'ester obtenu. Néanmoins, l'estérification enzymatique nécessite l'usage de solvants, source d'une éventuelle contamination du produit. Pour exemples, l'estérification peut être réalisée en présence de l'enzyme Corynbacterium sp avec l'acétone ou de l'enzyme R. arrhizus avec l'hexane.

### b- l'intérestérification

L'intérestérification est une réaction réversible qui consiste à chauffer des esters à 80-130°C en présence d'un catalyseur basique (0.1-0.3%) : cette réaction est très rapide.

R1 COOR2 + R3COOR4⇔ R1COOR4 + R3COOR2

### c- la transestérification

Durant une transestérification, le groupe alkoxyl de l'ester est remplacé par le groupe alkoxyl d'un alcool selon le schéma réactionnel suivant:

R1-COOR2 + R3-OH → R1-COOR3 + R2-OH

Cette réaction nécessite l'emploi d'un catalyseur basique et les températures réactionnelles varient généralement de 200 à 280°C.

Elle permet, par exemple, de former des monoglycérides à partir de glycérol et d'huile :

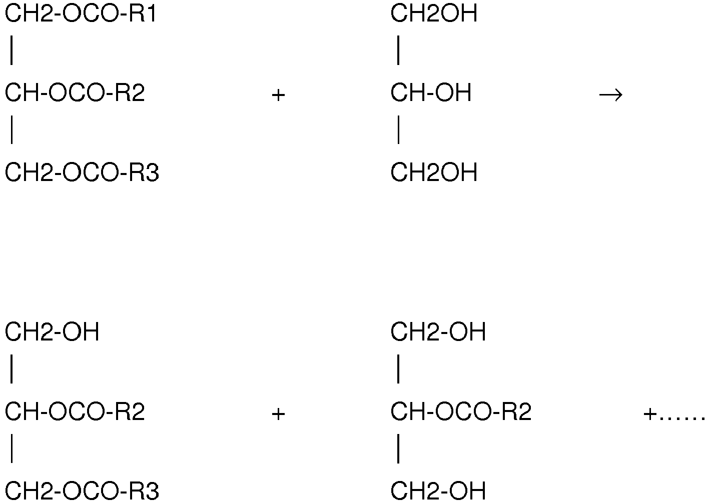

### Caractéristiques physico-chimiques des produits

Les esters sont caractérisés par un ensemble d'analyses chimiques. Parmi ces analyses, on peut citer l'indice d'acide, l'indice de peroxyde, l'indice de saponification, la teneur en eau, l'indice de réfraction...

Certaines analyses sont nécessaires pour l'identification de l'ester formé, alors que d'autres permettent davantage d'apprécier la stabilité du produit dans le temps.

L'indice d'acide, l'indice de peroxyde, la teneur en eau, l'indice d'anisidine sont des paramètres de stabilité.

L'indice d'acide et l'acidité rendent compte de l'altération hydrolytique des produits, en particulier les corps gras, et sont utiles pour apprécier leur qualité. Pour déterminer l'indice d'acide, il convient de se référer à la norme européenne NF EN ISO 660, juillet 1999.

L'indice de peroxyde rend compte de la formation des composés primaires d'oxydation : les peroxydes. Pour déterminer l'indice de peroxyde, il convient de se référer à la norme NF T 60-220, octobre 1995. Remarque : il existe des valeurs limites pour différents corps gras au-delà desquelles ils ne peuvent plus être considérés comme alimentaires.

L'indice d'anisidine constitue une mesure de la quantité d'aldéhydes présents dans les produits. En effet, l'oxydation spontanée des produits au contact de l'air donne naissance à de très nombreux dérivés oxygénés, en particulier les aldéhydes. Ceux-ci réagissent avec l'anisidine pour donner des composés colorés, dosés par absorbance dans les conditions d'essai spécifiées dans la norme européenne NF EN ISO 6885, décembre 2000.

La teneur en eau doit être la plus faible possible pour éviter l'altération hydrolytique des produits, en particulier des corps gras. Elle est déterminée selon différentes méthodes dont la méthode Karl Fischer décrite dans la norme NF ISO 8534, novembre 1996.

Pour garantir une bonne stabilité des produits, l'indice d'acide, l'indice de peroxyde, l'indice d'anisidine et la teneur en eau doivent être les plus faibles possibles.

### Rôle d'un tensio-actif non ionique

Les esters sont des composés très importants en cosmétique parce qu'ils représentent une forte proportion des matières premières utilisées dans les formulations.

Les esters jouent le rôle de :
- conditionneur,
- d'émollient en apportant de la souplesse à la peau,
- d'émulsionnant dans le cas d'émulsions,
- ou de solubilisant dans le cas de produits moussants.

Les émulsionnants et les solubilisants font partie de la famille des tensio-actifs non-ioniques.

Les tensio-actifs sont des molécules possédant au moins un groupement ayant une affinité pour l'eau (partie hydrophile) et un radical ayant une affinité pour l'huile (partie lipophile). Ils s'adsorbent aux interfaces et font chuter la tension superficielle en formant un film à la surface du liquide.

Il existe deux catégories de tensio-actifs :
- Les tensio-actifs ioniques (anionique, cationique et amphotère) qui possèdent une charge à leur surface, et qui sont donc agressifs et irritants pour la peau et les muqueuses.

Ces produits ne font pas l'objet de ce présent brevet.
- Les tensio-actifs non ioniques, appelés également émulsionnants pour les émulsions ou solubilisants pour les moussants, sont composés d'une chaîne grasse plus ou moins longue. Ce sont surtout des émulsionnants, des mouillants, des solubilisants.
Dans cette classe, on retrouve notamment les esters de sorbitan et les alcools gras polyoxyéthylénés.

La présente invention s'attache tout particulièrement à cette famille de tensio-actifs : produits non ioniques composés d'un groupe hydrophile et d'un groupe lipophile, à chaîne grasse plus ou moins longue.

### Fabrication d'esters

Les esters sont fabriqués selon des procédés classiques, connus par l'homme de l'art.

Le brevet N°621,104 déposé le 5 août 1942 aux USA par American Cyanamid Company consiste à produire des esters contenant des groupes hydroxyles libres en chauffant des polymères d'acides gras avec des polyalkylène-glycols ou des polyalkylène-oxydes en présence d'un catalyseur acide (acide sulfurique) pendant deux heures à 140 degrés. Ce procédé est assez court mais il utilise un catalyseur particulièrement délicat d'utilisation qu'il faut récupérer en fin de réaction.

Le brevet britannique N°439,435 consiste à produire des préparations lavantes contenant un ester de polyglycérols soluble, contenant des groupes hydroxyles libres. Il est indiqué que ces esters peuvent être produits par estérification partielle des polyglycérols avec des acides gras et il est aussi indiqué une méthode consistant à chauffer des graisses ou des huiles avec du glycérol en présence ou absence de catalyseur dans des conditions propices permettant de réaliser en une seule opération condensation et estérification. Dans ce cas, les temps de réaction restent particulièrement longs et risquent de dénaturer les chaînes grasses les plus fragiles.

Le brevet britannique 494,639 déposé par Herbert Schou en janvier 1938 propose de fabriquer un agent dispersant en estérifiant ou réestérifiant avec des polyglycérols des acides gras ou des esters d'acides gras polymérisés et oxydés et ce, en présence de catalyseurs tels que l'acétate de sodium, les sels de métal alcalins,.... L'estérification est effectuée à 260°C pendant 4-5h et la transestérification pendant 1h à 260°C. Ce procédé est néanmoins long car il demande de nombreuses étapes de séparation des catalyseurs et des produits n'ayant pas réagi.

Le brevet US5585506 déposé par Lonza en juillet 1994 'one phase production of polyglycerol esters' décrit un procédé de fabrication d'esters de polyglycérols. Ce procédé consiste à ajouter progressivement les acides gras (de 0.5 à 5 heures d'ajout et préférentiellement supérieur à 2 heures) et à estérifier ainsi les polyglycérols. La réaction se fait à 210-260°C sous 50-400mmHg, en présence d'un catalyseur basique (0.1 à 5%) préférentiellement pendant minimum 30-40 minutes après la fin de l'ajout. Ce procédé par ajout progressif reste long.

Le brevet GB 2 361 918 A, déposé par INTERPOLE LTD en novembre 2001, décrit un procédé de fabrication par chauffage diélectrique d'alcools polyhydroxylés partiellement ou totalement estérifiés à partir d'un mélange d'huiles végétales ou d'esters d'acides gras et de glycérol ou de saccharose.

La présente invention permet de réduire les temps réactionnels et de limiter les étapes de fabrication des esters.
De plus, les esters obtenus selon cette invention ont des caractéristiques physico-chimiques remarquables : cette invention permet de diminuer de manière significative leur indice d'acide et leur indice de peroxyde.

### Résumé de l'invention :

La présente invention concerne des procédés nouveaux d'estérification, transestérification et intérestérification selon la revendication 1.
Ces nouveaux procédés permettent de diminuer les temps réactionnels, ce qui représente un grand avantage industriel. De plus, ils conduisent à l'obtention d'esters aux caractéristiques physico-chimiques remarquables : ces procédés permettent de réduire de manière significative l'indice d'acide et l'indice de peroxyde du mélange.

Cette présente invention concerne un procédé d'estérification, transestérification et intérestérification caractérisé en ce qu'il comporte la fabrication par chauffage diélectrique d'alcools polyhydroxylés partiellement ou totalement estérifiés à partir d'un mélange d'huiles et graisses végétales, d'acides gras saturés ou insaturés, d'esters d'acides gras saturés ou insaturés, d'huiles et graisses animales, et de composés comportant des fonctions hydroxyles, dans laquelle les fréquences des ondes électromagnétiques utilisées dans le chauffage diélectrique varient de 3 MHz à 30 GHz et les réactifs comportant des fonctions hydroxyles sont choisis parmi les alcools, caractérisé en ce que, en tant qu'alcools, on utilise seul ou en mélange un ou des polyalcools primaires et/ou secondaires, choisis parmi les polyglycérols et les polyalkylèneglycols.

Le traitement thermique est effectué par chauffage diélectrique, de préférence dans une atmosphère dépourvue d'oxygène. Les fréquences des ondes électromagnétiques utilisées dans le chauffage diélectrique varient de 30 Ghz à 3 Mhz environ.

Il est aussi décrit les esters obtenus par le procédé selon l'invention et comportant un indice d'acide inférieur à 10 et préférentiellement inférieur à 2 et un indice de peroxyde inférieur à 20 et préférentiellement inférieur à 10 et préférentiellement inférieur à 5.

### Applications :

Les esters obtenus sont utilisés :
1- comme additifs alimentaires (fabrication de margarine, chocolat,...) ;
2- comme agents épaississants dans la fabrication de lubrifiants solubles dans l'eau ;
3- comme tensio-actifs non ioniques, émollients et hydratants dans les produits cosmétiques ;
4- comme agents anti-usure dans la fabrication de lubrifiants solubles dans l'eau ;
5- comme additifs dans la fabrication de plastiques (antistatiques,...).
Cette liste n'est pas exhaustive. Les esters d'alcools polyhydroxylés sont utilisables dans toutes les applications actuelles et futures de ces produits.

L'invention s'attache en particulier mais à titre non limitatif au traitement thermique d'huiles et graisses végétales, d'acides gras saturés ou insaturés, d'esters d'acides gras saturés ou insaturés, d'huiles et graisses animales, en proportions variables, avec des composés comportant des fonctions hydroxyles, de préférence dans une atmosphère dépourvue d'oxygène.

### Description détaillée de l'invention

La présente invention concerne des procédés nouveaux d'estérification, transestérification et intérestérification selon la revendication 1.

Les bénéfices de la présente invention sont :
1- de réduire de manière significative les temps de réaction ;
2- de réaliser la réaction en une seule étape ;
3- de ne pas utiliser de solvant ;
4- d'économiser de l'énergie (car les temps sont significativement plus courts) ;
5- d'éviter le burn-up et les sous-réactions indésirées ;
6- d'obtenir des esters avec des caractéristiques physico-chimiques remarquables.

On soumet les réactifs à des ondes électromagnétiques choisies dans les fréquences allant de environ 30 Ghz à environ 3 Mhz.

Le Demandeur a déposé une demande de brevet FR 0108906 relative à des applicateurs d'énergie adaptés au chauffage diélectrique, qui est applicable à la présente invention, ainsi que la demande de brevet FR 98 13770 et une demande de brevet PCT WO 00/26265 (PCT/FR99/02646) concernant un procédé original de chauffage diélectrique.

Ce procédé s'applique particulièrement à la préparation de ces alcools polyhydroxylés partiellement ou totalement estérifiés. L'homme du métier pourra s'y référer utilement pour les détails de mise en oeuvre.

Selon la présente invention, les procédés de transestérification, estérification et intérestérification suivent les paramètres de mise en oeuvre suivants :
1- Le chauffage est effectué par utilisation de fréquences micro-ondes.
2- Le chauffage est effectué par utilisation de hautes fréquences.
3- Il est mis en oeuvre avec ou sans catalyseurs.
4- On peut ajouter au réactif ou au mélange réactionnel des catalyseurs hétérogènes ou homogènes, et de préférence des catalyseurs basiques.
5- On peut ajouter au réactif ou au mélange réactionnel des catalyseurs répondant aux fréquences radio ou aux fréquences micro-ondes, tel que la montmorillonite.
6- Le réactif ou le mélange réactionnel, et éventuellement le(s) catalyseur(s), est placé dans un réacteur de type batch ou discontinu adapté pour recevoir des fréquences micro-ondes ou fréquences radio.
7- Le réactif ou le mélange réactionnel, et éventuellement le(s) catalyseur(s), est placé dans un réacteur adapté pour faire des réactions en continu.
8- Les fréquences micro-ondes MO sont comprises entre environ 300 MHz et environ 30 GHz, préférentiellement à 915 MHz (fréquence autorisée avec une tolérance de 1.4%) ou à 2.45 GHz (fréquence autorisée avec une tolérance de 2%).
9- Les hautes fréquences HF sont comprises entre environ 3 MHz et environ 300MHz, préférentiellement à 13.56 MHz (fréquence autorisée avec une tolérance de 0.05%) ou à 27.12 MHz (fréquence autorisée avec une tolérance de 0.6%)
10- La température à laquelle est soumis le réactif ou le mélange réactionnel, et éventuellement le(s) catalyseur(s), est comprise entre 50 et 400°C, préférentiellement entre 80 et 260°C. La température réactionnelle dépend des températures d'ébullition et/ou de dégradation des constituants du mélange.
11- Le temps de montée en température est choisi entre 3 et 180 minutes.
12- Le temps de réaction est compris entre 2 minutes et 15 heures, de préférence entre 2 minutes et 4 heures, de préférence encore entre 2 et 100 minutes. Ce temps de réaction dépend de l'ester que l'on souhaite obtenir, autrement dit du degré d'estérification attendu. Les alcools partiellement estérifiés nécessitent un temps réactionnel plus court que les alcools totalement estérifiés.
13 La quantité des réactifs introduits dans le milieu dépend du degré d'estérification que l'on souhaite atteindre et du nombre de fonctions hydroxyles OH présentes dans l'alcool utilisé.
   Plus le nombre de fonctions hydroxyles de l'ester formé est élevé, plus le produit est soluble dans l'eau mais sa stabilité dans le temps se voit réduite.
   De même, les esters à indice d'hydroxyle élevé sont utilisés dans les émulsions huile dans eau et les esters à indice d'hydroxyle faible sont préconisés pour les émulsions eau dans huile.
14 On effectue les réactions d'estérification, transestérification et intérestérification sous atmosphère normale ou riche en oxygène ou de préférence inerte ; éventuellement sous pression réduite, de préférence entre 6.66 et 1.33 kilopascal (50 et 10 mm de mercure) ; en renouvelant régulièrement l'atmosphère, sous agitation permanente.
15 On arrête les réactions d'estérification, transestérification et intérestérification en laissant refroidir ou en refroidissant le réactif ou le mélange réactionnel.

Le chauffage diélectrique, c'est-à-dire le chauffage sous fréquences micro-ondes ou hautes fréquences, comme décrit dans les brevets précités, permet des gains de temps et d'énergie, combinés à un coût d'investissement plus faible.

Ce gain de temps concerne à la fois la montée en température et le temps réactionnel. Il s'explique par la présence dans le milieu réactionnel d'un ou de plusieurs réactifs qui absorbent beaucoup les ondes électromagnétiques. En effet, pour la présente invention, certains réactifs, comportent des fonctions hydroxyles, soit les polyglycérols et les polyalkylèneglycols: ces produits ont des caractéristiques diélectriques élevées.

Les esters obtenus par le procédé selon l'invention présentent des caractéristiques physico-chimiques remarquables, c'est-à-dire qu'ils présentent à la fois un indice d'acide et un indice de peroxyde extrêmement faibles, voire nuls ce qui limite les dégradations des produits formés et conduit ainsi à des produits aux propriétés remarquables et surprenantes.

Les produits obtenus par ces procédés peuvent subir des traitements supplémentaires tels que l'hydrogénation, la décoloration, la désodorisation ou autres fonctionnalisations si ces traitements apportent des propriétés supplémentaires comme l'odeur, la couleur...

### Les réactifs

### a- Les réactifs pour la transestérification

Pour la présente invention, les réactifs peuvent être choisis parmi les huiles et graisses végétales, les esters d'acides gras saturés ou insaturés, les huiles et graisses animales et les composés comportant des fonctions hydroxyles.

En tant qu'huiles d'origine végétale, on peut mentionner, entre autres, l'huile de colza, l'huile de tournesol, l'huile d'arachide, l'huile d'olive, l'huile de noix, l'huile de maïs, l'huile de soja, l'huile de lin, l'huile de carthame, l'huile de noyaux d'abricot, l'huile d'amande douce, l'huile de chanvre, l'huile de pépins de raisin, l'huile de coprah, l'huile de palme, l'huile de graine de coton, l'huile de babassu, l'huile de jojoba, l'huile de sésame, l'huile d'argan, l'huile de chardon marie, l'huile de pépins de courge, l'huile de framboise, l'huile de Karanja, l'huile de Neem, l'huile d'oeillette, l'huile de noix du Brésil, l'huile de ricin, l'huile de ricin déshydratée, l'huile de noisette, l'huile de germe de blé, l'huile de bourrache, l'huile d'onagre, l'huile de Tung, l'huile de tall ou " tall oil ".

Ces huiles et graisses d'origine végétale, ainsi que leurs dérivés, peuvent subir un traitement préalable visant à les rendre plus réactifs ou au contraire moins réactifs. L'invention concerne aussi bien un réactif isolé qu'un mélange réactionnel comportant deux ou plusieurs composants. Ces mélanges réactionnels peuvent comporter des proportions équivalentes de chaque composant ou certains composants peuvent être majoritaires.

En tant qu'esters d'acides gras saturés ou insaturés, on peut utiliser seul ou en mélange et à titre d'exemples non limitatifs, un ou des esters obtenus par estérification entre un monoalcool et/ou polyol et au moins un acide gras saturé ou insaturé ; des cires ; des phospholipides ; des sphingolipides ; des glucolipides.

En tant qu'huiles et graisses d'origine animale, on peut citer entre autres, l'huile de cachalot, l'huile de dauphin, l'huile de baleine, l'huile de phoque, l'huile de sardine, l'huile de hareng, l'huile de squale, l'huile de foie de morue, l'huile de pied de boeuf, les graisses de porc, de cheval.

Les huiles et graisses peuvent subir un traitement préalable visant à les rendre plus réactives ou au contraire moins réactives comme par exemple l'hydrogénation, l'hydroxylation, l'époxydation, la phosphitation, la sulfonation.

Pour la présente invention, les réactifs comportant des fonctions hydroxyles sont choisis parmi les alcools.

En tant qu'alcools, on utilise seul ou en mélange, un ou des polyalcools primaires et/ou secondaires. Il s'agit des polyglycérols et des polyalkylèneglycols.

Parmi les catalyseurs ou adjuvants, on entendra à titre d'exemples non limitatifs les catalyseurs acides usuels (acide paratoluènesulfonique, acide sulfurique, acide phosphorique, acide perchlorique...), les catalyseurs basiques usuels (soude, potasse, alcoolate de métaux alcalins et de métaux alcalino-terreux, acétate de sodium, triéthylamines, dérivés de pyridine...), les résines acides et/ou basiques de type Amberlite™, Amberlyst™, Purolite™, Dowex™, Lewatit™, les zéolithes et les enzymes, les noirs de carbone et les fibres de carbone activées.

### b- les réactifs pour l'estérification

Pour la présente invention, les réactifs peuvent être choisis parmi les acides gras saturés ou insaturés et les composés comportant des fonctions hydroxyles.

En tant qu'acides gras saturés ou insaturés, on peut utiliser, seul ou en mélange, et à titre d'exemples non limitatifs, un ou des acides gras saturés comme l'acide palmitique, l'acide stéarique, un ou des acides gras monoinsaturés comme l'acide oléique, l'acide palmitoléique, l'acide myristique, l'acide pétrosélénique, l'acide érucique ; un ou des acides gras polyinsaturés comme par exemple l'acide linoléique, les acides alpha et gamma linolénique, l'acide arachidonique ; un ou des acides comprenant des diènes conjugués ou des triènes conjugués comme l'acide licanique, les isomères des acides linoléiques et linoléniques ; un ou des acides comprenant un ou plusieurs groupes hydroxyles comme l'acide ricinoléique.

Ces acides gras saturés ou insaturés, ainsi que leurs dérivés, peuvent subir un traitement préalable visant à les rendre plus réactifs ou au contraire moins réactifs, comme par exemple l'hydrogénation, l'hydroxylation, l'époxydation, la phosphitation, la sulfonation. L'invention concerne aussi bien un réactif isolé qu'un mélange réactionnel comportant deux ou plusieurs composants. Ces mélanges réactionnels peuvent comporter des proportions équivalentes de chaque composant ou certains composants peuvent être majoritaires.

Pour la présente invention, les réactifs comportant des fonctions hydroxyles sont choisis parmi les alcools.

En tant qu'alcools, on utilise seul ou en mélange, un ou des polyalcools primaires et/ou secondaires. Il s'agit des polyglycérols et des polyalkylèneglycols.

Parmi les catalyseurs ou adjuvants, on entendra à titre d'exemples non limitatifs les catalyseurs acides usuels (acide paratoluènesulfonique, acide sulfurique, acide phosphorique, acide perchlorique...), les catalyseurs basiques usuels (soude, potasse, alcoolate de métaux alcalins et de métaux alcalino-terreux, acétate de sodium, triéthylamines, dérivés de pyridine...), les résines acides et/ou basiques de type Amberlite™, Amberlyst™, Purolite™, Dowex™, Lewatit™, les zéolithes et les enzymes, les noirs de carbone et les fibres de carbone activées.

### c- les réactifs pour l'intérestérification

Pour la présente invention, les réactifs peuvent être choisis parmi les huiles et graisses végétales, les esters d'acides gras saturés ou insaturés et les huiles et graisses animales.

En tant qu'huiles d'origine végétale, on peut mentionner, entre autres, l'huile de colza, l'huile de tournesol, l'huile d'arachide, l'huile d'olive, l'huile de noix, l'huile de maïs, l'huile de soja, l'huile de lin, l'huile de carthame, l'huile de noyaux d'abricot, l'huile d'amande douce, l'huile de chanvre, l'huile de pépins de raisin, l'huile de coprah, l'huile de palme, l'huile de graine de coton, l'huile de babassu, l'huile de jojoba, l'huile de sésame, l'huile d'argan, l'huile de chardon marie, l'huile de pépins de courge, l'huile de framboise, l'huile de Karanja, l'huile de Neem, l'huile d'oeillette, l'huile de noix du Brésil, l'huile de ricin, l'huile de ricin déshydratée, l'huile de noisette, l'huile de germe de blé, l'huile de bourrache, l'huile d'onagre, l'huile de Tung, l'huile de tall ou " tall oil ".

Ces huiles et graisses d'origine végétale peuvent subir un traitement préalable visant à les rendre plus réactives ou au contraire moins réactives. L'invention concerne aussi bien un réactif isolé qu'un mélange réactionnel comportant deux ou plusieurs composants. Ces mélanges réactionnels peuvent comporter des proportions équivalentes de chaque composant ou certains composants peuvent être majoritaires.

En tant qu'esters d'acides gras saturés ou insaturés, on peut utiliser seul ou en mélange et à titre d'exemples non limitatifs, un ou des esters obtenus par estérification entre un monoalcool et/ou polyol et au moins un acide gras saturé ou insaturé ; des cires ; des phospholipides ; des sphingolipides ; des glucolipides.

En tant qu'huiles et graisses d'origine animale, on peut citer entre autres, l'huile de cachalot, l'huile de dauphin, l'huile de baleine, l'huile de phoque, l'huile de sardine, l'huile de hareng, l'huile de squale, l'huile de foie de morue, l'huile de pied de boeuf, les graisses de porc, de cheval.

Les huiles et graisses peuvent subir un traitement préalable visant à les rendre plus réactifsves ou au contraire moins réactifsves comme par exemple l'hydrogénation, l'hydroxylation, l'époxydation, la phosphitation, la sulfonation.

Parmi les catalyseurs ou adjuvants, on entendra à titre d'exemples non limitatifs les catalyseurs acides usuels (acide paratoluènesulfonique, acide sulfurique, acide phosphorique, acide perchlorique...), les catalyseurs basiques usuels (soude, potasse, alcoolate de métaux alcalins et de métaux alcalino-terreux, acétate de sodium, triéthylamines, dérivés de pyridine...), les résines acides et/ou basiques de type Amberlite™, Amberlyst™, Purolite™, Dowex™, Lewatit™, les zéolithes et les enzymes, les noirs de carbone et les fibres de carbone activées.

Des exemples concrets mais non limitatifs de l'invention vont être maintenant présentés.

### EXEMPLES

Les exemples suivants permettent de mettre en évidence les gains de temps et la qualité des esters obtenus grâce à ces nouveaux procédés d'estérification, transestérification et intérestérification.

Les exemples suivants sont réalisés à une fréquence de 2,45 Ghz ou de 915 MHz.

### I- Diminution des temps réactionnels grâce au chauffage diélectrique

Comme indiqué dans ce présent brevet, le chauffage diélectrique, c'est à dire le chauffage sous fréquences micro-ondes ou hautes fréquences, permet des gains de temps et d'énergie, combinés à un coût d'investissement plus faible.

Les tableaux ci-dessous montrent l'efficacité du chauffage diélectrique comparé au chauffage classique.

### a- transestérification

| **réactifs** | | **Ester (2)** | **AcNa⁽³⁾** | **T(°C) (8)** | **t(MO) (4)** | **t(CL) (5)** |
|---|---|---|---|---|---|---|
| **PEG⁽⁶⁾** | | | | | | |
| huile | PEG300 | mono | 0.5% | 220°C | 15 min | >4h |
| huile | PEG400 | mono | 0.5% | 220°C | 15min | >4h |
| huile | PEG600 | mono | 0.5% | 220°C | 15min | >4h |
| huile | PEG1500 | excès | 0.5% | 280°C | 2h40 | >8h |

| **POLYGLYCEROL ⁽⁷⁾** | | | | | | |
|---|---|---|---|---|---|---|
| huile | Polyglyceryl-6 | di | 0.5% | 220°C | 40min | 5h |
| huile | Polyglyceryl-2 | di | 0.25% | 260°C | 2min | 1h⁽¹⁾ |

### Remarques :

(1) : cet essai fait référence au brevet GB 494,639 de Schou, cité dans la présente demande.
(2) : les alcools polyhydroxylés peuvent être plus ou moins estérifiés.
   a. Mono : formation de monoesters d'alcools
   b. Di : formation de diesters d'alcools
   c. Excès : l'huile est en large excès par rapport à l'alcool.
(3) : AcNa : Acétate de sodium, catalyseur basique
(4) : t(MO) : temps réactionnel sous chauffage micro-onde
(5) : t(CL) : temps réactionnel sous chauffage classique
(6) : PEG : Polyéthylèneglycol de masse moléculaire 300, 400, 600 ou 1500, d'où les abréviations respectives PEG300, PEG400, PEG600 et PEG1500.
(7) POLYGLYCEROL-n : condensation de n molécules de glycérol, produit majoritaire
(8) T(°C) température en °C.

Ces deux exemples d'alcools (PEG, Polyglycérol,) mettent en évidence le gain de temps obtenu en opérant par chauffage diélectrique.

### b-estérification

| **réactifs** | | **Ester⁽²⁾** | **AcNa⁽³⁾** | **T(°C)** | **t(MO)⁽⁴⁾** | **t(CL)⁽⁵⁾** |
|---|---|---|---|---|---|---|
| **POLYGLYCEROL ⁽⁷⁾** | | | | | | |
| Acide oléique | Polyglyceryl-6 | di | 0.25% | 230°C | 2h10 | 4h30⁽⁶⁾ |
| Acide stéarique | Polyglyceryl-2 | tri | 0.25% | 260°C | 1h40 | 4-5h⁽¹⁾ |

### Légendes:

(1) : cet essai fait référence au brevet GB 494,639 de Schou, cité dans la présente demande.
(2) : les alcools polyhydroxylés peuvent être plus ou moins estérifiés.
   a. Di : formation de diesters d'alcools
   b. Tri: formation de triesters d'alcools
(3) : AcNa : Acétate de sodium, catalyseur basique
(4) : t(MO) : temps réactionnel sous chauffage micro-onde
(5) : t(CL) : temps réactionnel sous chauffage classique
(6) POLYGLYCEROL-n : condensation de n molécules de glycérol, produit majoritaire

Ces deux exemples mettent en évidence le gain de temps obtenu en opérant par chauffage diélectrique.

### II- Qualité des esters obtenus

Les esters selon l'invention présentent des caractéristiques physico-chimiques remarquables, c'est-à-dire qu'ils présentent à la fois un indice d'acide et un indice de peroxyde extrêmement faibles, voire nuls ce qui limite les dégradations des produits formés et conduit à des produis aux propriétés remarquables et surprenantes.

Les tableaux ci-dessous montrent la qualité des esters obtenus par chauffage diélectrique.

### a- transestérification

| **Huiles** | **Alcool** | **Ester** | **T°C** | **t(MO) min** | **Cat %** | **IAi** | **IAf** | **IPi** | **IPf** |
|---|---|---|---|---|---|---|---|---|---|
| **PEG** | | | | | | | | | |
| noisette | PEG400 | mono | 220 | 15 | 0.5 | 0.63 | 0.08 | 21.06 | 0 |
| neem | PEG600 | di | 220 | 15 | 0.5 | 5.05 | 1.80 | 20.62 | 0 |
| karanja | PEG600 | di | 220 | 15 | 0.5 | 7.27 | 1.80 | 88.29 | 0 |
| karanja | PEG600 | mono | 220 | 15 | 0.5 | 4.94 | 1.32 | 60.04 | 0 |
| bourrache | PEG600 | mono | 220 | 15 | 0.5 | 1.22 | 0.19 | 40.20 | 1.98 |
| onagre | PEG600 | mono | 220 | 15 | 0.5 | 1.0 | 0.62 | 50.3 | 0 |

| **POLYGLYCEROL POL-n** | | | | | | | | | |
|---|---|---|---|---|---|---|---|---|---|
| carthame | Pol-6 | di | 220 | 40 | 0.5 | 0.6 | 0.19 | 15.8 | 2.10 |
| amande douce | Pol-6 | di | 220 | 40 | 0.5 | 0.86 | 0.18 | 10.3 | 1.10 |
| noisette | Pol-6 | di | 220 | 40 | 0.5 | 1.02 | 0.18 | 29.9 | 1.50 |
| olive | Pol-6 | di | 220 | 40 | 0.5 | 1.16 | 0.19 | 13.0 | 1.34 |

### Légendes:

IAi = indice d'acide du mélange initial, exprimé en mg KOH/g
IAf = indice d'acide final (de l'ester), exprimé en mg KOH/g
IPi= indice de peroxyde du mélange initial, exprimé en µéquiO2/kg
IPf= indice de peroxyde final (de l'ester), exprimé en µéquiO2/kg
Cata = catalyseur basique
t(MO) = temps réactionnel par chauffage diélectrique
Ester = les alcools polyhydroxylés peuvent être plus ou moins estérifiés. Di : formation de diesters d'alcools
POLYGLYCEROL-n = Pol-n : condensation de n molécules de glycérol, produit majoritaire

### b- estérification

| **Acides gras** | **Alcool** | **Ester** | **T°C** | **t(MO)** | **Cat %** | **IAi** | **IAf** |
|---|---|---|---|---|---|---|---|
| **POLYGLYCEROL POL-n** | | | | | | | |
| Acide oléique | Pol-6 | di | 230 | 2h10 | 0.25 | 157 | 0.5 |
| Acide stéarique | Pol-2 | tri | 260 | 1h40 | 0.25 | 155 | 2.2 |

### Légendes:

IAi = indice d'acide du mélange initial, exprimé en mg KOH/g
IAf = indice d'acide final (de l'ester), exprimé en mg KOH/g
IPi= indice de peroxyde du mélange initial, exprimé en µéquiO2/kg
IPf= indice de peroxyde final (de l'ester), exprimé en µéquiO2/kg
Cata = catalyseur basique
t(MO) = temps réactionnel par chauffage diélectrique
Ester = les alcools polyhydroxylés peuvent être plus ou moins estérifiés.
   Di : formation de diesters d'alcools
   Tri : formation de triesters d'alcools
POLYGLYCEROL-n = Pol-n : condensation de n molécules de glycérol, produit majoritaire
PEG 400, PEG 600 : Polyéthylèneglycol de masse moléculaire respective 400,600.

## Revendications

1. Procédé d'estérification, transestérification et intérestérification **caractérisé en ce qu'**il comporte la fabrication par chauffage diélectrique d'alcools polyhydroxylés partiellement ou totalement estérifiés à partir d'un mélange d'huiles et graisses végétales, d'acides gras saturés ou insaturés, d'esters d'acides gras saturés ou insaturés, d'huiles et graisses animales, et de composés comportant des fonctions hydroxyles, dans laquelle les fréquences des ondes électromagnétiques utilisées dans le chauffage diélectrique varient de 3 MHz à 30 GHz et les réactifs comportant des fonctions hydroxyles sont choisis parmi les alcools, **caractérisé en ce que**, en tant qu'alcools, on utilise seul ou en mélange un ou des polyalcools primaires et/ou secondaires, choisis parmi les polyglycérols et les polyalkylèneglycols.

2. Procédé selon la revendication 1 **caractérisé en ce que** le chauffage diélectrique est réalisé de préférence dans une atmosphère dépourvue d'oxygène.

3. Procédé selon la revendication 1 ou 2 **caractérisé en ce que** les fréquences desdites ondes électromagnétiques sont des fréquences micro-ondes MO comprises entre 300 MHz et 30 GHz, préférentiellement à 915MHz qui représente une fréquence autorisée avec une tolérance de 1.4% ou à 2.45GHz qui représente une fréquence autorisée avec une tolérance de 2%.

4. Procédé selon la revendication 1 ou 2 **caractérisé en ce que** les fréquences desdites ondes électromagnétiques sont des hautes fréquences HF comprises entre environ 3 MHz et environ 300MHz, préférentiellement à 13.56 MHz qui représente une fréquence autorisée avec une tolérance de 0.05% ou à 27.12 MHz qui représente une fréquence autorisée avec une tolérance de 0.6%.

5. Procédé selon l'une quelconque des revendications 1 à 4 **caractérisé en ce que** la température à laquelle est soumis le réactif ou le mélange réactionnel, et éventuellement le(s) catalyseur(s), est comprise entre 50 et 400°C, préférentiellement entre 80 et 260°C.

6. Procédé selon l'une quelconque des revendications 1 à 5 **caractérisé en ce que** le temps de montée en température est choisi entre 3 et 180 minutes.

7. Procédé selon l'une quelconque des revendications 1 à 6 **caractérisé en ce que** le temps de réaction est compris entre 2 minutes et 15 heures, de préférence entre 2 minutes et 4 heures, et de manière encore plus préférée encore entre 2 et 100 minutes.

8. Procédé selon l'une quelconque des revendications 1 à 7 **caractérisé en ce qu'**il est mis en oeuvre avec ou sans catalyseurs.

9. Procédé selon l'une quelconque des revendications 1 à 8 **caractérisé en ce qu'**on ajoute au réactif ou au mélange réactionnel des catalyseurs hétérogènes ou homogènes, et de préférence des catalyseurs basiques.

10. Procédé selon la revendication 9 **caractérisé en ce qu'**on ajoute au réactif ou au mélange réactionnel des catalyseurs répondant aux fréquences radio ou aux fréquences micro-ondes, tel que la montmorillonite.

11. Procédé selon l'une quelconque des revendications 1 à 10 **caractérisé en ce que** le réactif ou le mélange réactionnel, et éventuellement le(s) catalyseur(s), est placé dans un réacteur de type batch ou discontinu adapté pour recevoir des fréquences micro-ondes ou fréquences radio.

12. Procédé selon l'une quelconque des revendications 1 à 11 **caractérisé en ce que** le réactif ou le mélange réactionnel, et éventuellement le(s) catalyseur(s), est placé dans un réacteur adapté pour faire des réactions en continu.

13. Procédé selon l'une quelconque des revendications 1 à 12 **caractérisé en ce qu'**il comporte une estérification ou transestérification ou intérestérification sous atmosphère normale ou riche en oxygène ou de préférence inerte ; sous pression réduite, de préférence entre 50 et 10 mm de mercure ; en renouvelant régulièrement l'atmosphère, sous agitation permanente.

14. Procédé selon la revendication 13 **caractérisé en ce qu'**il comporte une étape d'arrêt de la réaction d'estérification, de transestérification et d'intérestérification en laissant refroidir ou en refroidissant le réactif ou le mélange réactionnel.

15. Procédé selon l'une quelconque des revendications 1 à 14 **caractérisé en ce que** les réactifs sont choisis parmi les huiles et graisses végétales.

16. Procédé selon la revendication 15 **caractérisé en ce que**, en tant qu'huiles d'origine végétale, on utilise l'huile de colza, l'huile de tournesol, l'huile d'arachide, l'huile d'olive, l'huile de noix, l'huile de maïs, l'huile de soja, l'huile de lin, l'huile de carthame, l'huile de noyaux d'abricot, l'huile d'amande douce, l'huile de chanvre, l'huile de pépins de raisin, l'huile de coprah, l'huile de palme, l'huile de graine de coton, l'huile de babassu, l'huile de jojoba, l'huile de sésame, l'huile d'argan, l'huile de chardon marie, l'huile de pépins de courge, l'huile de framboise, l'huile de Karanja, l'huile de Neem, l'huile d'oeillette, l'huile de noix du Brésil, l'huile de ricin, l'huile de ricin déshydratée, l'huile de noisette, l'huile de germe de blé, l'huile de bourrache, l'huile d'onagre, l'huile de Tung, l'huile de tall ou "tall oil ".

17. Procédé selon la revendication 14 ou 15 **caractérisé en ce que** ces huiles et graisses d'origine végétale, ainsi que leurs dérivés, subissent un traitement préalable visant à les rendre plus réactifs ou au contraire moins réactifs.

18. Procédé selon l'une quelconque des revendications 1 à 17 **caractérisé en ce qu'**on utilise un réactif isolé ou un mélange réactionnel comportant deux ou plusieurs composants, ces mélanges réactionnels comportant des proportions équivalentes de chaque composant ou certains composants peuvent être majoritaires.

19. Procédé selon l'une quelconque des revendications 1 à 18 **caractérisé en ce que**, en tant qu'acides gras saturés ou insaturés, on utilise, seul ou en mélange, un ou des acides saturés comme l'acide palmitique ou l'acide stéarique, un ou des acides gras monoinsaturés comme l'acide oléique, l'acide palmitoléique, l'acide myristique, l'acide pétrosélénique, l'acide érucique ; un ou des acides gras polyinsaturés comme par exemple l'acide linoléique, les acides alpha et gamma linolénique, l'acide arachidonique ; un ou des acides comprenant des diènes conjugués ou des triènes conjugués comme l'acide licanique, les isomères des acides linoléiques et linoléniques ; un ou des acides comprenant un ou plusieurs groupes hydroxyles comme l'acide ricinoléique.

20. Procédé selon l'une quelconque des revendications 1 à 19 **caractérisé en ce que**, en tant qu'esters d'acides gras saturés ou insaturés, on utilise seul ou en mélange un ou des esters obtenus par estérification entre un monoalcool et/ou polyol et au moins un acide gras saturé ou insaturé ; des cires ; des phospholipides ; des sphingolipides ; des glucolipides.

21. Procédé selon l'une quelconque des revendications 1 à 20 **caractérisé en ce qu'**on utilise des huiles et les graisses d'origine animale choisies parmi l'huile de cachalot, l'huile de dauphin, l'huile de baleine, l'huile de phoque, l'huile de sardine, l'huile de hareng, l'huile de squale, l'huile de foie de morue, l'huile de pied de boeuf, les graisses de porc, de cheval.

22. Procédé selon l'une quelconque des revendications 1 à 21 **caractérisé en ce que**, parmi les catalyseurs ou adjuvants, on utilise les catalyseurs acides usuels (acide paratoluènesulfonique, acide sulfurique, acide phosphorique, acide perchlorique), les catalyseurs basiques usuels (soude, potasse, alcoolate de métaux alcalins et de métaux alcalino-terreux, acétate de sodium, triéthylamines, dérivés de pyridine), les résines acides et/ou basiques de type Amberlite™, Amberlyst™, Purolite™, Dowex™, Lewatit™, les zéolithes et les enzymes, les noirs de carbone et les fibres de carbone activées.

23. Procédé selon l'une quelconque des revendications 1 à 22 **caractérisé en ce que**, à une fréquence de 2,45 Ghz ou de 915 MHz : il comporte une
**transestérification**
| **réactifs** | | **Ester (2)** | **AcNa (3)** | **T(°C) (8)** | **t(MO) (4)** | **t(CL) (5)** |
|---|---|---|---|---|---|---|
| **PEG⁽⁶⁾** | | | | | | |
| huile | PEG300 | mono | 0.5% | 220°C | 15 min | >4h |
| huile | PEG400 | mono | 0.5% | 220°C | 15min | >4h |
| huile | PEG600 | mono | 0.5% | 220°C | 15min | >4h |
| huile | PEG1500 | excès | 0.5% | 280°C | 2h40 | >8h |
| **POLYGLYCEROL ⁽⁷⁾** | | | | | | |
|---|---|---|---|---|---|---|
| huile | Polyglyceryl-6 | di | 0.5% | 220°C | 40min | 5h |
| huile | Polyglyceryl-2 | di | 0.25% | 260°C | 2min | 1h⁽¹⁾ |
Remarques :
(1) cet essai fait référence au brevet **GB 494,639** de Schou, cité dans la présente demande.
(2) les alcools polyhydroxylés peuvent être plus ou moins estérifiés.
a Mono : formation de monoesters d'alcools
b Di : formation de diesters d'alcools
c Excès : l'huile est en large excès par rapport à l'alcool.
(3) AcNa : Acétate de sodium, catalyseur basique
(4) t(MO) : temps réactionnel sous chauffage micro-onde
(5) t(CL) : temps réactionnel sous chauffage classique
(6) PEG : Polyéthylèneglycol de masse moléculaire 300, 400, 600 ou 1500, d'où les abréviations respectives PEG300, PEG400, PEG600 et PEG1500.
(7) POLYGLYCEROL-n : condensation de n molécules de glycérol, produit majoritaire
(8) T(°C) température en °C.

24. Procédé selon l'une quelconque des revendications 1 à 22 **caractérisé en ce que**, à une fréquence de 2,45 GHz ou de 915 MHz : Avec
**estérification**
| **réactifs** | | **Ester⁽²⁾** | **AcNa⁽³⁾** | **T(°C)** | **t(MO)⁽⁴⁾** | **t(CL)⁽⁵⁾** |
|---|---|---|---|---|---|---|
| **POLYGLYCEROL ⁽⁷⁾** | | | | | | |
| Acide oléique | Polyglyceryl-6 | di | 0.25% | 230°C | 2h10 | 4h30⁽⁶⁾ |
| Acide stéarique | Polyglyceryl-2 | tri | 0.25% | 260°C | 1h40 | 4-5h⁽¹⁾ |
Légendes:
(1) cet essai fait référence au brevet GB 494,639 de Schou, cité dans la présente demande.
(2) les alcools polyhydroxylés peuvent être plus ou moins estérifiés.
a. Di : formation de diesters d'alcools
b. Tri : formation de triesters d'alcools
(3) AcNa : Acétate de sodium, catalyseur basique
(4) t(MO) : temps réactionnel sous chauffage micro-onde
(5) t(CL) : temps réactionnel sous chauffage classique
(6) POLYGLYCEROL-n : condensation de n molécules de glycérol, produit majoritaire

25. Procédé selon l'une quelconque des revendications 1 à 22 **caractérisé en ce que**
**a- transestérification**
| **Huiles** | **Alcool** | **Ester** | **T°C** | **t(MO) min** | **Cat %** | **IAi** | **IAf** | **IPi** | **IPf** |
|---|---|---|---|---|---|---|---|---|---|
| **PEG** | | | | | | | | | |
| noisette | PEG400 | mono | 220 | 15 | 0.5 | 0.63 | 0.08 | 21.06 | 0 |
| neem | PEG600 | di | 220 | 15 | 0.5 | 5.05 | 1.80 | 20.62 | 0 |
| karanja | PEG600 | di | 220 | 15 | 0.5 | 7.27 | 1.80 | 88.29 | 0 |
| karanja | PEG600 | mono | 220 | 15 | 0.5 | 4.94 | 1.32 | 60.04 | 0 |
| bourrache | PEG600 | mono | 220 | 15 | 0.5 | 1.22 | 0.19 | 40.20 | 1.98 |
| onagre | PEG600 | mono | 220 | 15 | 0.5 | 1.0 | 0.62 | 50.3 | 0 |
| **POLYGLYCEROL POL-n** | | | | | | | | | |
|---|---|---|---|---|---|---|---|---|---|
| carthame | Pol-6 | di | 220 | 40 | 0.5% | 0.6 | 0.19 | 15.8 | 2.10 |
| amande douce | Pol-6 | di | 220 | 40 | 0.5% | 0.86 | 0.18 | 10.3 | 1.10 |
| noisette | Pol-6 | di | 220 | 40 | 0.5% | 1.02 | 0.18 | 29.9 | 1.50 |
| olive | Pol-6 | di | 220 | 40 | 0.5% | 1.16 | 0.19 | 13.0 | 1.34 |
Légendes:
IAi = indice d'acide du mélange initial, exprimé en mg KOH/g
IAf = indice d'acide final (de l'ester), exprimé en mg KOH/g
IPi= indice de peroxyde du mélange initial, exprimé en µéquiO2/kg
IPf= indice de peroxyde final (de l'ester), exprimé en µéquiO2/kg
Cata = catalyseur basique
t(MO) = temps réactionnel par chauffage diélectrique
Ester = les alcools polyhydroxylés peuvent être plus ou moins estérifiés.
Di : formation de diesters d'alcools
POLYGLYCEROL-n = Pol-n : condensation de n molécules de glycérol, produit majoritaire
**b- estérification**
| **Acides gras** | **Alcool** | **Ester** | **T°C** | **t(MO)** | **Cata** | **IAi** | **IAf** |
|---|---|---|---|---|---|---|---|
| **POLYGLYCEROL POL-n** | | | | | | | |
| Acide oléique | Pol-6 | di | 230 | 2h10 | 0.25% | 157 | 0.5 |
| Acide stéarique | Pol-2 | tri | 260 | 1h40 | 0.25% | 155 | 2.2 |
Légendes:
IAi = indice d'acide du mélange initial, exprimé en mg KOH/g
IAf = indice d'acide final (de l'ester), exprimé en mg KOH/g
IPi= indice de peroxyde du mélange initial, exprimé en µéquiO2/kg
IPf= indice de peroxyde final (de l'ester), exprimé en µéquiO2/kg
Cata = catalyseur basique
t(MO) = temps réactionnel par chauffage diélectrique
Ester = les alcools polyhydroxylés peuvent être plus ou moins estérifiés.
Di : formation de diesters d'alcools
Tri : formation de triesters d'alcools
POLYGLYCEROL-n = Pol-n : condensation de n molécules de glycérol, produit majoritaire
PEG 400,PEG 600 :Polyéthylèneglycol de masse moléculaire respective 400,600.

## Patentansprüche

1. Verfahren zur Veresterung und Umesterung, **dadurch gekennzeichnet, dass** es die Erzeugung ganz oder teilweise veresterter polyhydroxylierter Alkohole mittels dielektrischer Erwärmung aus einer Mischung aus Pflanzenölen und -fetten, gesättigten oder ungesättigten Fettsäuren, gesättigten oder ungesättigten Fettsäureestern, Ölen und Fetten tierischen Ursprungs und Zusammensetzungen mit Hydroxylfunktionen, wobei die Frequenzen der bei der dielektrischen Erwärmung verwendeten elektromagnetischen Wellen zwischen 3 MHz und 30 GHz variieren und die Reagenzien mit Hydroxylfunktionen aus den Alkoholen gewählt sind, **dadurch gekennzeichnet, dass** als Alkohole mindestens ein primärer und/oder sekundärer Polyalkohol allein oder in einer Mischung verwendet wird, der aus den Polyglycerinen und den Polyalkylenglykolen gewählt ist.

2. Verfahren nach Anspruch 1, **dadurch gekennzeichnet, dass** die dielektrische Erwärmung vorzugsweise in einer sauerstofffreien Atmosphäre erfolgt.

3. Verfahren nach Anspruch 1 oder 2, **dadurch gekennzeichnet, dass** die Frequenzen der elektromagnetischen Wellen Mikrowellenfrequenzen MO zwischen 300 MHz und 30 GHz sind, und vorzugsweise 915 MHz, was eine erlaubte Frequenz mit einer Toleranz von 1,4 % ist, oder 2,45 GHz, was eine erlaubte Frequenz mit einer Toleranz von 2 % ist, betragen.

4. Verfahren nach Anspruch 1 oder 2, **dadurch gekennzeichnet, dass** die Frequenzen der elektromagnetischen Wellen Hochfrequenzen HF zwischen etwa 3 MHz und etwa 300 MHz sind, und vorzugsweise 13,56 MHz, was eine erlaubte Frequenz mit einer Toleranz von 0,05% ist, oder 27,12 MHz, was eine erlaubte Frequenz mit einer Toleranz von 0,6% ist, betragen.

5. Verfahren nach einem der Ansprüche 1 - 4, **dadurch gekennzeichnet, dass** die Temperatur, der das Reagenz bzw. die Reaktionsmischung und ggf. der/die Katalysator(en) ausgesetzt sind, zwischen 50 und 400 °C, vorzugsweise zwischen 80 und 260 °C, liegt.

6. Verfahren nach einem der Ansprüche 1 - 5, **dadurch gekennzeichnet, dass** die Erwärmungszeit zwischen 3 und 180 min gewählt ist.

7. Verfahren nach einem der Ansprüche 1 - 6, **dadurch gekennzeichnet, dass** die Reaktionszeit zwischen 2 min und 15 h, vorzugsweise zwischen 2 min und 4 h, insbesondere zwischen 2 und 100 min, liegt.

8. Verfahren nach einem der Ansprüche 1 - 7, **dadurch gekennzeichnet, dass** es mit oder ohne Katalysatoren ausgeführt wird.

9. Verfahren nach einem der Ansprüche 1 - 8, **dadurch gekennzeichnet, dass** dem Reagenz bzw. der Reaktionsmischung hetero- oder homogene Katalysatoren, vorzugsweise alkalische Katalysatoren, hinzugegeben werden.

10. Verfahren nach Anspruch 9, **dadurch gekennzeichnet, dass** dem Reagenz bzw. der Reaktionsmischung Katalysatoren hinzugegeben werden, die auf Radio- oder Mikrowellenfrequenzen reagieren, wie z.B. Montmorillonit.

11. Verfahren nach einem der Ansprüche 1 - 10, **dadurch gekennzeichnet, dass** das Reagenz bzw. die Reaktionsmischung und ggf. der/die Katalysator(en) in einen Chargenreaktor oder einen diskontinuierlichen Reaktor platziert werden, der dazu geeignet ist, Mikrowellen- oder Radiofrequenzen zu empfangen.

12. Verfahren nach einem der Ansprüche 1 - 11, **dadurch gekennzeichnet, dass** das Reagenz bzw. die Reaktionsmischung und ggf. der/die Katalysator(en) in einen Reaktor platziert werden, der dazu geeignet ist, kontinuierliche Reaktionen durchzuführen.

13. Verfahren nach einem der Ansprüche 1 - 12, **dadurch gekennzeichnet, dass** es eine Ver- oder Umesterung unter normaler oder sauerstoffreicher oder vorzugsweise unter einer inerten Atmosphäre unter reduziertem Druck, vorzugsweise zwischen 50 und 10 mmHg, unter regelmäßiger Auffrischung der Atmosphäre und dauerhaftem Rühren umfasst.

14. Verfahren nach Anspruch 13, **dadurch gekennzeichnet, dass** es einen Schritt des Stoppens der Ver- und Umesterungsreaktion umfasst, wobei man das Reagenz bzw. die Reaktionsmischung abkühlt oder abkühlen lässt.

15. Verfahren nach einem der Ansprüche 1 - 14, **dadurch gekennzeichnet, dass** es sich bei den Reagenzien um Pflanzenöle und -fette handelt.

16. Verfahren nach Anspruch 15, **dadurch gekennzeichnet, dass** als Pflanzenöle Rapsöl, Sonnenblumenöl, Erdnussöl, Olivenöl, Nussöl, Maisöl, Sojaöl, Leinöl, Safloröl, Aprikosensamenöl, süsses Mandelöl, Hanföl, Traubenkernöl, Kokosnussöl, Palmöl, Baumwollsaatöl, Babassuöl, Jojobaöl, Sesamöl, Arganöl, Mariendistelöl, Kürbiskernöl, Himbeeröl, Karanjaöl, Neemöl, Mohnöl, Paranussöl, Rizinusöl, dehydriertes Rizinusöl, Haselnussöl, Weizenkeimöl, Borretschsamenöl, Nachtkerzenöl, Tung-Öl oder Tallöl verwendet wird.

17. Verfahren nach Anspruch 14 oder 15, **dadurch gekennzeichnet, dass** diese Pflanzenöle und -fette sowie ihre Derivate einer Vorbehandlung unterzogen werden, um ihre Reaktionsfähigkeit zu erhöhen oder zu reduzieren.

18. Verfahren nach einem der Ansprüche 1 - 17, **dadurch gekennzeichnet, dass** ein isoliertes Reagenz oder eine Reaktionsmischung verwendet wird, umfassend mindestens zwei Bestandteile, wobei diese Reaktionsmischungen alle Bestandteile in gleichen Anteilen umfassen oder bestimmte Bestandteile die Mehrheit darstellen können.

19. Verfahren nach einem der Ansprüche 1 - 18, **dadurch gekennzeichnet, dass** als gesättigte oder ungesättigte Fettsäuren, allein oder in einer Mischung, verwendet werden: mindestens eine gesättigte Säure wie z.B. Palmitinsäure oder Stearinsäure, mindestens eine einfach ungesättigte Fettsäure wie z.B. Ölsäure, Palmitoleinsäure, Myristinsäure, Petroselensäure, Erucasäure; mindestens eine mehrfach ungesättigte Fettsäure wie z.B. Linolsäure, Alpha- und Gamma-Linolsäure, Arachidonsäure; mindestens eine Säure, die konjugierte Di- oder Triene umfasst, wie z.B. Licansäure, Linol- und Linolensäureisomere; mindestens eine Säure, die mindestens eine Hydroxylgruppe umfasst, wie z.B. Ricinolsäure.

20. Verfahren nach einem der Ansprüche 1 - 19, **dadurch gekennzeichnet, dass** als gesättigte oder ungesättigte Fettsäureester, allein oder in einer Mischung, verwendet werden: mindestens ein Ester, der durch Veresterung zwischen einem Monoalkohol und/oder Polyol und mindestens einer gesättigten oder ungesättigten Fettsäure entsteht; Wachse, Phospholipide, Sphingolipide, Glycolipide.

21. Verfahren nach einem der Ansprüche 1 - 20, **dadurch gekennzeichnet, dass** Öle und Fette tierischen Ursprungs verwendet werden, die gewählt sind aus: Pottwalöl, Delphinöl, Walöl, Seehundöl, Sardinenöl, Heringsöl, Haifischöl, Lebertran, Rindsfussöl, Schweinefette, Hundefette.

22. Verfahren nach einem der Ansprüche 1 - 21, **dadurch gekennzeichnet, dass** aus den Katalysatoren oder Hilfsstoffe verwendet werden: die üblichen sauren Katalysatoren (P-Toluolsulfonsäure, Schwefelsäure, Phosphorsäure, Perchlorsäure), die üblichen alkalischen Katalysatoren (Natronlauge, Kalilauge, Alkalimetall- und Erdalkalimetallalkoholate, Natriumacetat, Triethylamine, Pyridinderivate), saure und/oder alkalische Harze der Art Amberlite™, Amberlyst™, Purolite™, Dowex™, Lewatit™, Zeolithen, Ruße und aktivierte Kohlenstofffasern.

23. Verfahren nach einem der Ansprüche 1 - 22, **dadurch gekennzeichnet, dass** es bei einer Frequenz von 2,45 GHz oder 915 MHz umfasst:
eine Umesterung
| Reagenzien | | Ester ⁽²⁾ | AcNa ⁽³⁾ | T(°C) ⁽⁸⁾ | t(MO) ⁽⁴⁾ | t(CL) ⁽⁵⁾ |
|---|---|---|---|---|---|---|
| PEG⁽⁶⁾ | | | | | | |
| Öl | PEG300 | mono | 0.5% | 220°C | 15 min | >4h |
| Öl | PEG400 | mono | 0.5% | 220°C | 15min | >4h |
| Öl | PEG600 | mono | 0.5% | 220°C | 15min | >4h |
| Öl | PEG1500 | Überschuss | 0.5% | 280°C | 2 h 40 min | >8 h |
| POLYGLYCERIN ⁽⁷⁾ | | | | | | |
|---|---|---|---|---|---|---|
| Öl | Polyglyceryl-6 | di | 0.5% | 220°C | 40min | 5h |
| Öl | Polyglyceryl-2 | di | 0.25% | 260°C | 2min | 1 h⁽¹⁾ |
Bemerkungen:
(1) bei diesem Versuch wird auf die in der vorliegenden Anmeldung zitierte Patentschrift GB 494,639 an Schou Bezug genommen.
(2) Die polyhydroxylierten Alkohole können mehr oder weniger verestert sein.
a Mono : Bildung von Alkoholmonoestern
b Di : Bildung von Alkoholdiestern
c Überschuss : Es gibt einen starken Ölüberschuss gegenüber dem Alkohol
(3) AcNa : Natriumacetat, alkalischer Katalysator
(4) t(MO) : Reaktionszeit unter Mikrowellenerwärmung
(5) t(CL) : Reaktionszeit unter herkömmlicher Erwärmung
(6) PEG: Polyethylenglykol mit Molekulargewicht 300, 400, 600 oder 1500, daher die jeweiligen Abkürzungen PEG300, PEG400, PEG600 bzw. PEG1500.
(7) POLYGLYCERIN-n : Kondensation von n Glycerinmolekülen, Hauptprodukt
(8) T(°C) Temperatur in °C.

24. Verfahren nach einem der Ansprüche 1 - 22, **dadurch gekennzeichnet, dass** bei einer Frequenz von 2,45 GHz oder 915 MHz:
mit **Veresterung**
| Reagenzien | | Ester ⁽²⁾ | AcNa ⁽³⁾ | T(°C) | t(MO)⁽⁴⁾ | t(CL)⁽⁵⁾ |
|---|---|---|---|---|---|---|
| POLYGLYCERIN ⁽⁷⁾ | | | | | | |
| Ölsäure | Polyglyceryl-6 | di | 0.25% | 230 °C | 2 h 10 min | 4h 30min ⁽⁶⁾ |
| Stearinsäure | Polyglyceryl-2 | tri | 0.25% | 260 °C | 1 h 40 min | 4-5h⁽¹⁾ |
Legenden:
(1) bei diesem Versuch wird auf die in der vorliegenden Anmeldung zitierte Patentschrift GB 494,639 an Schou Bezug genommen.
(2) Die polyhydroxylierten Alkohole können mehr oder weniger verestert sein.
a. Di: Bildung von Alkoholdiestern
b. Tri: Bildung von Alkoholtriestern
(3) AcNa : Natriumacetat, alkalischer Katalysator
(4) t(MO) : Reaktionszeit unter Mikrowellenerwärmung
(5) t(CL) : Reaktionszeit unter herkömmlicher Erwärmung
(7) POLYGLYCERIN-n : Kondensation von n Glycerinmolekülen, Hauptprodukt

25. Verfahren nach einem der Ansprüche 1 - 22, **dadurch gekennzeichnet, dass**
**a- Umesterung**
| Öle | Alkohol | Ester | T°C | t(MO) min | Kat % | IAi | IAf | IPi | IPf |
|---|---|---|---|---|---|---|---|---|---|
| PEG | | | | | | | | | |
| Haselnuss | PEG400 | mono | 220 | 15 | 0.5 | 0.63 | 0.08 | 21.06 | 0 |
| Neem | PEG600 | di | 220 | 15 | 0.5 | 5.05 | 1.80 | 20.62 | 0 |
| Karanja | PEG600 | di | 220 | 15 | 0.5 | 7.27 | 1.80 | 88.29 | 0 |
| Karanja | PEG600 | mono | 220 | 15 | 0.5 | 4.94 | 1.32 | 60.04 | 0 |
| Borretsch | PEG600 | mono | 220 | 15 | 0.5 | 1.22 | 0.19 | 40.20 | 1.98 |
| Nachtkerze | PEG600 | mono | 220 | 15 | 0.5 | 1.0 | 0.62 | 50.3 | 0 |
| POLYGLYCERIN POL-n : | | | | | | | | | |
|---|---|---|---|---|---|---|---|---|---|
| Färberdistel | Pol-6 | di | 220 | 40 | 0.5% | 0.6 | 0.19 | 15.8 | 2.10 |
| Süßmandel | Pol-6 | di | 220 | 40 | 0.5% | 0.86 | 0.18 | 10.3 | 1.10 |
| Haselnuss | Pol-6 | di | 220 | 40 | 0.5% | 1.02 | 0.18 | 29.9 | 1.50 |
| Olive | Pol-6 | di | 220 | 40 | 0.5% | 1.16 | 0.19 | 13.0 | 1.34 |
Legenden:
IAi = Säurewert der Ausgangsmischung in mg KOH/g
IAf = Endsäurewert (des Esters) in mg KOH/g
IPi = Peroxidzahl der Ausgangsmischung in µEqO2/kg
IPf = Endperoxidzahl (des Esters) in µEqO2/kg
Kata = Alkalischer Katalysator
t(MO) = Reaktionszeit unter dielektrischer Erwärmung
Ester = Die polyhydroxylierten Alkohole können mehr oder weniger verestert sein.
Di: Bildung von Alkoholdiestern
POLYGLYCERIN-n = Pol-n : Kondensation von n Glycerinmolekülen, Hauptprodukt
**b- Veresterung**
| **Fettsäuren** | **Alkohol** | **Ester** | **T°C** | **t(MO)** | **Kata** | **IAi** | **IAf** |
|---|---|---|---|---|---|---|---|
| **POLYGLYCERIN POL-n :** | | | | | | | |
| Ölsäure | Pol-6 | di | 230 | 2h10 | 0.25% | 157 | 0,5 |
| Stearinsä ure | Pol-2 | tri | 260 | 1 h40 | 0.25% | 155 | 2,2 |
Legenden:
IAi = Säurewert der Ausgangsmischung in mg KOH/g
IAf = Endsäurewert (des Esters) in mg KOH/g
IPi = Peroxidzahl der Ausgangsmischung in µEqO2/kg
IPf = Endperoxidzahl (des Esters) in µEqO2/kg
Kata = Alkalischer Katalysator
t(MO) = Reaktionszeit unter dielektrischer Erwärmung
Ester = Die polyhydroxylierten Alkohole können mehr oder weniger verestert sein.
Di: Bildung von Alkoholdiestern
Tri: Bildung von Alkoholtriestern
POLYGLYCERIN-n = Pol-n : Kondensation von n Glycerinmolekülen, Hauptprodukt
PEG 400, PEG 600: Polyethylenglykol, jeweils mit dem Molekulargewicht 400 bzw. 600.

## Claims

1. Method for esterification, transesterification and interesterification **characterized in that** it comprises the dielectric heating production of polyhydroxylated alcohols that are partially or totally esterified from a mixture of vegetable oils and fats, saturated or unsaturated fatty acids, saturated or unsaturated fatty acid esters, animal oils and fats, and compounds having hydroxyl functions, wherein the frequencies of the electromagnetic waves used in the dielectric heating range from 3 MHz to 30 GHz, and the reagents having hydroxyl functions are chosen from among alcohols, **characterized in that**, as alcohols, one or more primary and/or secondary polyalcohols chosen from among polyglycerols and polyalkylene glycols, are used alone or as a mixture.

2. Method according to claim 1 **characterized in that** the dielectric heating is preferably carried out in an oxygen-free atmosphere.

3. Method according to claim 1 or 2 **characterized in that** the frequencies of the electromagnetic waves are MW microwave frequencies between 300 MHz and 30 GHz, preferably 915 MHz which represents an authorized frequency with a tolerance of 1.4% or 2.45 GHz, which represents an authorized frequency with a tolerance of 2%.

4. Method according to claim 1 or 2 **characterized in that** the frequencies of the electromagnetic waves are HF high frequencies between about 3 MHz and about 300 MHz, preferably 13.56 MHz, which represents an authorized frequency with a tolerance of 0.05% or 27.12. MHz which represents an authorized frequency with a tolerance of 0.6%.

5. Method according to any one of the claims 1 to 4 **characterized in that** the temperature to which the reagent or the reaction mixture and optionally the catalyst(s) is/are subjected is between 50 and 400°C, preferably between 80 and 260°C.

6. Method according to any one of the claims 1 to 5 **characterized in that** the temperature rise time is chosen between 3 and 180 minutes.

7. Method according to any one of the claims 1 to 6 **characterized in that** the reaction time is between 2 minutes and 15 hours, preferably between 2 minutes and 4 hours, and even more preferably between 2 and 100 minutes.

8. Method according to any one of the claims 1 to 7 **characterized in that** it is implemented with or without catalysts.

9. Method according to any one of the claims 1 to 8, **characterized in that** heterogeneous or homogeneous catalysts, and preferably basic catalysts, are added to the reagent or to the reaction mixture.

10. Method according to claim 9, **characterized in that** catalysts responding to radio frequencies or microwave frequencies, such as montmorillonite, are added to the reagent or to the reaction mixture.

11. Method according to any one of the claims 1 to 10, **characterized in that** the reagent or the reaction mixture, and optionally the catalyst(s), is/are placed in a batch or batch-type reactor intended to receive microwave frequencies or radio frequencies.

12. Method according to any one of the claims 1 to 11 **characterized in that** the reagent or the reaction mixture, and optionally the catalyst(s), is/are placed in a reactor intended to effect reactions continuously.

13. Method according to any one of the claims 1 to 12 **characterized in that** it comprises esterification or transesterification or interesterification in a normal atmosphere or rich in oxygen or preferably inert; under reduced pressure, preferably between 50 and 10 mm of mercury; by regularly renewing the atmosphere, with constant stirring.

14. Method according to claim 13 **characterized in that** it comprises a step of stopping the esterification, transesterification or interesterification reaction by cooling, or allowing to cool, the reagent or the reaction mixture.

15. Method according to any one of the claims 1 to 14, **characterized in that** the reagents are vegetable oils and fats.

16. Method according to claim 15, **characterized in that**, as oils of plant origin, are used rapeseed oil, sunflower oil, peanut oil and olive oil, walnut oil, corn oil, soybean oil, linseed oil, safflower oil, apricot kernel oil, sweet almond oil, hemp oil, grape seed oil, coconut oil, palm oil, cottonseed oil, babassu oil, jojoba oil, sesame oil, olive oil, argan oil, milk thistle oil, pumpkin seed oil, raspberry oil, karanja oil, neem oil, flaxseed oil, Brazil nut oil, castor oil, dehydrated castor oil, hazelnut oil, wheat germ oil, borage oil, evening primrose oil, tung oil, tall oil.

17. Method according to claim 14 or 15 **characterized in that** these oils and fats of plant origin, and their derivatives, undergo a prior treatment to make them more reactive or, alternatively, less reactive.

18. Method according to any one of the claims 1 to 17, **characterized in that** an isolated reagent or reaction mixture comprising two or more components is used, wherein these reaction mixtures comprise equivalent proportions of each component, or certain components may be the majority.

19. Method according to any one of the claims 1 to 18 **characterized in that**, as saturated or unsaturated fatty acids, are used, alone or in mixture, one or more saturated acids such as palmitic acid or stearic acid, or on of the monounsaturated fatty acid(s) such as oleic acid, palmitoleic acid, myristic acid, petroselenic acid, erucic acid; one or more polyunsaturated fatty acids, for example linoleic acid, alpha and gamma linolenic acids, arachidonic acid; acid(s) comprising conjugated dienes or conjugated trienes such as licanic acid, the isomers of linoleic and linolenic acids; acid(s) comprising one or more hydroxyl groups such as ricinoleic acid.

20. Method according to any one of the claims 1 to 19, **characterized in that**, as saturated or unsaturated fatty acid esters, are used one or more esters obtained by esterification between a monoalcohol and/or polyol and at least one saturated or unsaturated fatty acid; waxes; phospholipids; sphingolipids; glucolipids.

21. Method according to any one of the claims 1 to 20 **characterized in that** oils and fats of animal origin are selected from among sperm whale oil, dolphin oil, whale oil, seal oil, sardine oil, herring oil, shark oil, cod liver oil, beef foot oil, pork fat, horse fat.

22. Method according to any one of the claims 1 to 21, **characterized in that**, among catalysts or adjuvants, are used, the usual acid catalysts (para-toluenesulphonic acid, sulfuric acid, phosphoric acid, perchloric acid), the usual basic catalysts (sodium hydroxide alkali metal and alkaline earth metal alcoholates, sodium acetate, triethylamines, pyridine derivatives), acidic and/or basic resins of the Amberlite™, Amberly™, Purolite™, Dowex™, Lewati™ type, zeolites and enzymes, carbon blacks and activated carbon fibers.

23. Method according to any one of the claims 1 to 22 **characterized in that**, at a frequency of 2.45 GHz or 915 MHz: there is
**transesterification**
| reagents | | Ester (2) | AcNa (3) | T(°C) (8) | t(MO) (4) | t(CL) (5) |
|---|---|---|---|---|---|---|
| PEG ⁽⁶⁾ | | | | | | |
| oil | PEG300 | mono | 0.5% | 220°C | 15 min | >4h |
| oil | PEG400 | mono | 0.5% | 220°C | 15min | >4h |
| oil | PEG600 | mono | 0.5% | 220°C | 15min | >4h |
| oil | PEG1500 | Excess | 0.5% | 280°C | 2 h 40 min | >8 h |
| POLYGLYCERIN ⁽⁷⁾ | | | | | | |
|---|---|---|---|---|---|---|
| oil | Polyglyceryl-6 | di | 0.5% | 220°C | 40min | 5h |
| oil | Polyglyceryl-2 | di | 0.25% | 260°C | 2min | 1 h⁽¹⁾ |
Comments:
(1) this test refers to GB Patent 494,639 Schou, cited in the present application.
(2) polyhydric alcohols may be more or less esterified.
a. Mono: formation of monoesters of alcohols
b. Di: formation of alcohol diesters
c. Excess: the oil is in large excess compared to alcohol.
(3) AcNa: Sodium acetate, basic catalyst
(4) t(MO): reaction time under microwave heating
(5) t(CL): reaction time under conventional heating
(6) PEG: Polyethylene glycol of molecular weight 300, 400, 600 or 1500, hence the respective abbreviations PEG300, PEG400, PEG600 and PEG1500.
(7) POLYGLYCEROL-n: condensation of n glycerol molecules, majority product
(8) T(°C) temperature in °C.

24. Method according to any one of the claims 1 to 22, **characterized in that** at a frequency of 2.45 GHz or 915 MHz, there is
**esterification**
| reagents | | Ester ⁽²⁾ | AcNa ⁽³⁾ | T(°C) | t(MO)⁽⁴⁾ | t(CL)⁽⁵⁾ |
|---|---|---|---|---|---|---|
| POLYGLYCERIN ⁽⁷⁾ | | | | | | |
| Oleic acid | Polyglyceryl-6 | di | 0.25% | 230 °C | 2 h 10 min | 4h 30min ⁽⁶⁾ |
| Stearic acid | Polyglyceryl-2 | tri | 0.25% | 260 °C | 1 h 40 min | 4-5h⁽¹⁾ |
Captions:
(1) This test refers to GB Patent 494,639 of Schou, cited in the present application.
(2) polyhydric alcohols may be more or less esterified.
a. Di: formation of alcohol diesters
b. Tri: formation of triesters of alcohols
(3) AcNa: Sodium acetate, basic catalyst
(4) t(MO): reaction time under microwave heating
(5) t(CL): reaction time under conventional heating
(7) POLYGLYCEROL-n: condensation of n glycerol molecules, majority product

25. Method according to one of the claims 1 to 22, **characterized in that** **a- transesterification**
| Oils | Alcohol | Ester | T°C | t(MO) min | Cat % | IAi | IAf | IPi | IPf |
|---|---|---|---|---|---|---|---|---|---|
| PEG | | | | | | | | | |
| Hazelnut | PEG400 | mono | 220 | 15 | 0.5 | 0.63 | 0.08 | 21.06 | 0 |
| Neem | PEG600 | di | 220 | 15 | 0.5 | 5.05 | 1.80 | 20.62 | 0 |
| Karanja | PEG600 | di | 220 | 15 | 0.5 | 7.27 | 1.80 | 88.29 | 0 |
| Karanja | PEG600 | mono | 220 | 15 | 0.5 | 4.94 | 1.32 | 60.04 | 0 |
| Borage | PEG600 | mono | 220 | 15 | 0.5 | 1.22 | 0.19 | 40.20 | 1.98 |
| E. primrose oil | PEG600 | mono | 220 | 15 | 0.5 | 1.0 | 0.62 | 50.3 | 0 |
| POLYGLYCERIN POL-n : | | | | | | | | | |
|---|---|---|---|---|---|---|---|---|---|
| Safflower | Pol-6 | di | 220 | 40 | 0.5% | 0.6 | 0.19 | 15.8 | 2.10 |
| Sweet almond | Pol-6 | di | 220 | 40 | 0.5% | 0.86 | 0.18 | 10.3 | 1.10 |
| Hazelnut | Pol-6 | di | 220 | 40 | 0.5% | 1.02 | 0.18 | 29.9 | 1.50 |
| Olive | Pol-6 | di | 220 | 40 | 0.5% | 1.16 | 0.19 | 13.0 | 1.34 |
Captions:
IAi = acid number of the initial mixture, expressed in mg KOH/g
IAf = final acid number (of the ester), expressed in mg KOH/g
IPi = peroxide value of the initial mixture, expressed in µequi02/kg
IPf = final peroxide value (of the ester), expressed in µequi02/kg
Cata = basic catalyst
t(MO) = reaction time by dielectric heating
Ester = the polyhydric alcohols may be more or less esterified.
Di: formation of alcohol diesters
POLYGLYCEROL-n = Pol-n: condensation of n molecules of glycerol, majority product
**b- esterification**
| Fatty acids | Alcohol | **Ester** | **T°C** | **t(MO)** | **Cata** | **IAi** | **IAf** |
|---|---|---|---|---|---|---|---|
| **POLYGLYCERIN POL-n :** | | | | | | | |
| Oleic acid | Pol-6 | di | 230 | 2h10 | 0.25% | 157 | 0,5 |
| Stearic acid | Pol-2 | tri | 260 | 1 h40 | 0.25% | 155 | 2,2 |
Captions:
IAi = acid number of the initial mixture, expressed in mg KOH/g
IAf = final acid number (of the ester), expressed in mg KOH/g
IPi = peroxide value of the initial mixture, expressed in µequi02/kg
IPf = final peroxide value (of the ester), expressed in µequi02/kg
Cata = basic catalyst
t(MO) = reaction time by dielectric heating
Ester = the polyhydric alcohols may be more or less esterified.
Di: formation of alcohol diesters
Tri:: formation of alcohol triesters
POLYGLYCEROL-n = Pol-n: condensation of n molecules of glycerol, majority product
PEG 400, PEG 600: Polyethylene glycol of respective molecular weight 400, 600.
